# FASCICULE DE BREVET EUROPEEN

(11) **EP 0 936 897 B1**
(45) Date de publication et mention de la délivrance du brevet: **20.11.2002**
(21) Numéro de dépôt: 97928340.5
(22) Date de dépôt: 12.06.1997
(51) Int. Cl.: A61K 7/42, A61K 7/48

(54) **COMPOSITION A ACTIVITE BRONZANTE ET PHOTOPROTECTRICE ET SES APPLICATIONS ESTHETIQUES**
LICHTSCHUTZ- UND BRÄUNUNGSMITTEL UND SEINE ÄSTHETISCHE VERWENDUNG
COMPOSITION HAVING TANNING AND SUN-SCREENING ACTIVITY, AND COSMETIC USES THEREOF

(30) Priorité: 12.06.1996 FR 9607279; 07.01.1997 FR 9700082
(43) Date de publication de la demande: 25.08.1999
(73) Titulaire: Laboratoire Oenobiol, 75016 Paris (FR)
(72) Inventeur: HARANG, Benoît, F-92310 Sevres (FR)
(74) Mandataire: Vialle-Presles, Marie José
(86) Numéro de dépôt international: FR9701052
(87) Numéro de publication internationale: WO97047278

(56) Documents cités:
- EP-A- 0 467 795
- EP-A- 0 748 625
- WO-A-85/03226
- WO-A-94/06310
- DE-A- 19 503 604
- FR-A- 2 100 886
- FR-A- 2 320 732
- FR-A- 2 339 403
- GB-A- 2 274 235
- US-A- 5 290 605

## Description

La présente invention est relative à une composition à activité bronzante et photoprotectrice destinée à être administrée soit par voie orale, soit par application topique ainsi qu'à ses applications esthétiques.

La pigmentation cutanée des mammifères, en général, et de l'Homme en particulier, repose sur la biosynthèse d'un pigment azoté, la mélanine, à partir de la tyrosine, dont la régulation est sous l'influence de plusieurs paramètres :
- la tyrosinase, produite par la cellule pigmentaire,
- le mélanocyte, qui sous l'influence de la lumière ultraviolette, catalyse l'oxydation de la tyrosine en dihydroxyphénylalanine (DOPA), puis en dihydroxyindole et enfin en mélanine,
- la mélanine peut ensuite subir des polymérisations oxydatives qui vont accentuer sa coloration ; ce pigment est présent sous forme d'organites, les mélanosomes, dans les dendrites des mélanocytes précités et ces mélanosomes sont ensuite transmis aux kératinocytes, qui vont transporter la mélanine jusqu'à la surface de la peau où elle sera éliminée progressivement lors de la desquamation naturelle.

La mélanine formée est composée de deux types, (1) l'eu-mélanine, qui est d'une couleur brun-noir et se forme par polymérisation de produits d'oxydation de la dopaquinone et (2) la phéo-mélanine, qui est d'une couleur brun-roux et se forme par polymérisation de dérivés soufrés de la dopaquinone ; seuls les premiers ont un effet photoprotecteur.

La couleur de la peau et son intensité dépendent donc du contenu des kératinocytes en mélanine, du type de mélanine présente dans ces kératinocytes, de la vitesse de la desquamation et de l'épaisseur de la couche cornée qui est la couche contenant le plus de pigment.

Essentiellement pour des raisons esthétiques, l'Homme s'expose de plus en plus au soleil, pour avoir une peau bronzée.

Or les radiations ultraviolettes (UVA et UVB), habituellement proposées pour bronzer (lampes à bronzer par exemple), présentent des dangers aussi bien à court terme qu'à long terme.

En outre, en raison d'une diminution progressive de la couche d'ozone, les effets délétères des radiations ultraviolettes solaires, nécessitent de renforcer la photoprotection des individus.

Pour combattre les effets néfastes à court terme des radiations ultraviolettes, à savoir l'érythème solaire et la photosensibilisation, il est nécessaire de protéger la peau.

Toutefois, l'ensemble des produits à administration orale proposés dans l'art antérieur, sont uniquement axés sur l'effet esthétique : obtention d'une peau « bronzée » et comprennent l'administration de β-carotène à des doses nettement supérieures à celles conseillées dans la Directive CEE 90/496, à savoir 800 µg/jour, exprimé en vitamine A, soit 6 mg de β-carotène/jour.

Aux doses habituellement utilisées dans ces produits de l'art antérieur, c'est-à-dire de l'ordre de 12 à 30 mg/j, il y a accumulation de β-carotène dans les kératinocytes, qui donne à la peau une apparence de « bronzage », mais ne permet pas d'avoir en outre une photoprotection active par induction d'eu-mélanine, lors de l'exposition ultérieure au soleil.

En conséquence, la Demanderesse s'est notamment donné pour but de pourvoir à une composition apte à être administrée par voie orale, qui réponde mieux aux besoins de la pratique que les compositions de l'art antérieur, notamment en ce:
- qu'elle présente un pouvoir bronzant à des doses journalières en β-carotène, significativement inférieures à celles antérieurement utilisées à cet effet et ne dépassant pas les limites préconisées dans la Directive précitée, et
- qu'elle présente, à la fois un pouvoir bronzant et un effet photoprotecteur actif, du fait qu'elle induit notamment la production d'eu-mélanine.

La présente invention a pour objet une composition à visée esthétique, régulatrice de la pigmentation cutanée et adaptée aussi bien à l'administration par voie orale qu'à l'application sur la peau, du type comprenant des caroténoïdes, caractérisée en ce qu'elle comprend essentiellement un composant (a) constitué d'au moins un caroténoïde d'origine naturelle à activité provitaminique A sélectionné dans le groupe constitué par le β-carotène et l'α-carotène ou un mélange de ceux-ci et un composant (b), correspondant à au moins un caroténoïde d'origine naturelle sans activité provitaminique A, constitué par du lycopène, éventuellement associé à un autre composant (b), sélectionnée dans le groupe constitué par la zéaxanthine, la cryptoxanthine et la lutéine ou un mélange de ceux-ci, les composants (a) et le lycopène étant dans un rapport compris entre 0,95/1 et 1/50.

On entend par régulateur de la pigmentation, au sens de la présente invention, un principe actif à pouvoir bronzant, c'est-à-dire induisant la synthèse endogène de mélanines.

De manière surprenante, dans des rapport composant (a)/lycopène inférieurs à 1, aussi bien la composition destinée à une administration par voie orale que la composition destinée à être administrée sur la peau présentent à la fois un pouvoir bronzant sans exposition au soleil et un effet photoprotecteur, lors d'une exposition ultérieure au soleil.

Alors que la Demanderesse a trouvé, de manière surprenante que la composition destinée à une administration par voie orale, à des doses journalières en β-carotène significativement inférieures à celles antérieurement utilisées, présente un pouvoir bronzant et un effet photoprotecteur lorsque ledit rapport (a)/lycopène est inférieur à 1 et de préférence compris entre 0,95/1 et 1/50, il n'y a pas de limitation de doses pour les compositions aptes à être administrées par la voie topique, qui comprennent les mêmes composants (a) et lycopène, dans les mêmes rapports que ceux définis pour les compositions administrées par voie orale.

Selon un autre mode de réalisation avantageux de ladite composition, lorsqu'elle est destinée à être administrée par voie orale, ladite composition comprend le composant (a) à une dose inférieure ou égale à 800 µg, exprimée en vitamine A, de préférence à une dose de 400 µg, exprimée en vitamine A. [→ un peu moins de 3 mg de composant (a)].

Selon une disposition avantageuse de ce mode de réalisation, le composant (b) comprend du lycopène à une dose comprise entre 1 et 3 mg.

Selon un autre mode de réalisation avantageux de ladite composition elle comprend en outre des vitamines et/ou des oligo-éléments.

De telles compositions ont un effet inducteur de bronzage important (augmentation de la synthèse endogène de mélanines) sans provoquer de caroténodermie et présentent également une efficacité photoprotectrice (érythème solaire).

Selon un autre mode de réalisation avantageux de ladite composition elle comprend en outre au moins un excipient approprié, adapté soit à l'administration orale, soit à l'administration topique.

Selon un autre mode de réalisation avantageux de ladite composition, elle se présente sous la forme de capsules molles.

De préférence, pour l'obtention de l'effet esthétique recherché, une telle composition est administrée à raison de deux unités/jour.

Selon un autre mode de réalisation avantageux de ladite composition, elle se présente sous la forme de crème, de lotion, de liposomes ou de gel.

La description comprend en outre des exemples de mise en oeuvre de la présente invention, ainsi que des dessins annexés, dans lesquels :

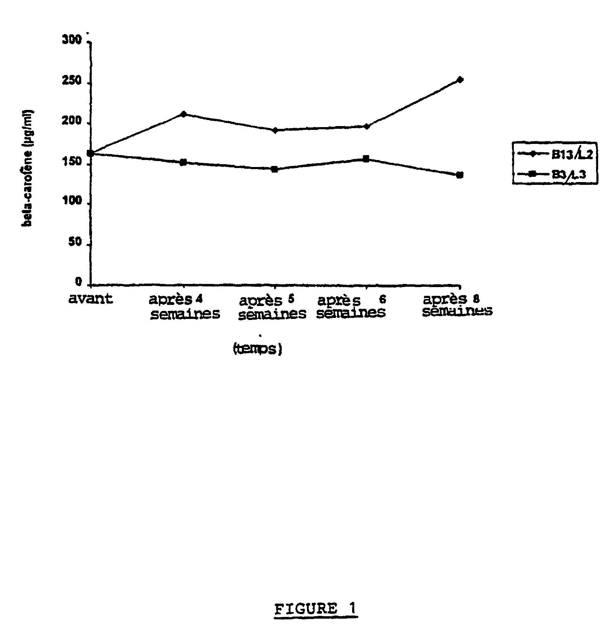

- la figure 1 illustre la variation du β-carotène dans la peau après administration orale d'une composition selon l'invention (composant (a) [β-carotène ou B] 2,86 mg et composant (b) [lycopène ou L] : 3 mg), dénommée ci-après composition B3/L3 comparée à une composition comprenant 13 mg de composant (a) et 2 mg de composant (b), dénommée B13/L2 ; cette figure comporte en abscisse le temps en semaine et en ordonnée la concentration en β-carotène dans la peau en µg/ml ;

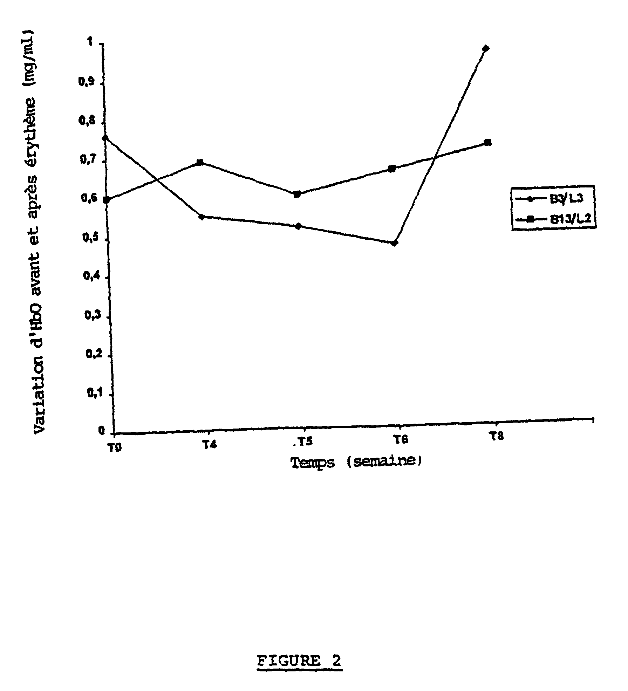

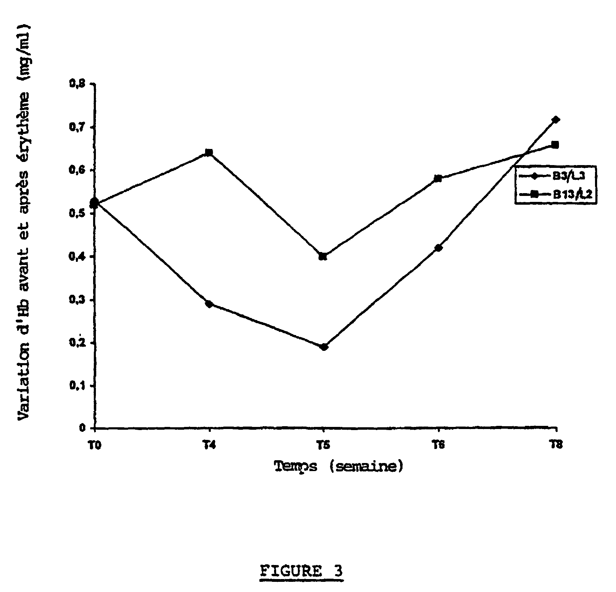

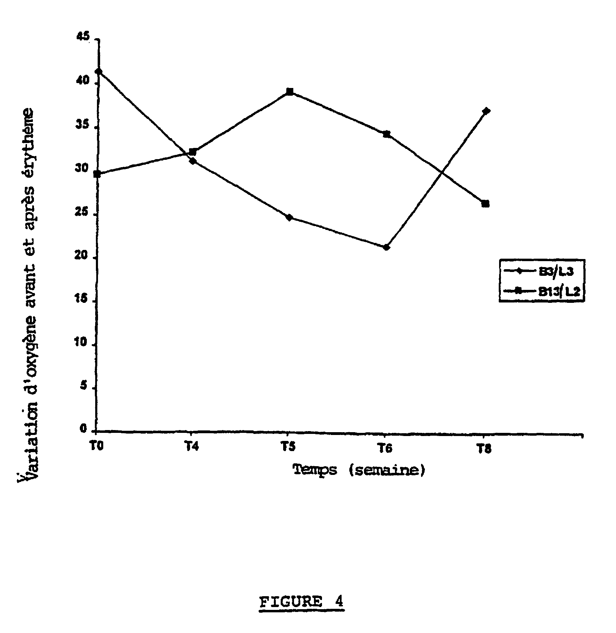

- les figures 2, 3 et 4 illustrent l'évolution de l'érythème après administration orale d'une composition selon l'invention (composant (a) [β-carotène ou B] 2,86 mg et composant (b) [lycopène ou L] : 3 mg), dénommée ci-après composition B3/L3 comparée à une composition comprenant 13 mg de composant (a) et 2 mg de composant (b), dénommée B13/L2 ; la figure 2 comprend en abscisse le temps en semaine et en ordonnée la variation de l'hémoglobine oxydée (HbO) avec et sans érythème, en mg/ml ; la figure 3 comprend en abscisse le temps en semaine et en ordonnée la variation de l'hémoglogine (Hb) avec et sans érythème, en mg/ml et la figure 4 comprend en abscisse le temps en semaine et en ordonnée la variation d'O₂ avec et sans érythème, en mg/ml.

### Exemple 1 : Préparation d'une composition à administration orale selon l'invention

| - Capsules molles contenant : | |
|---|---|
| | B3/L3 |
| **Excipients :** | |
| . Huile de soja hydrogénée | 40 mg |
| . Huile de blé | 95 mg |
| . Lécithine de soja | 20 mg |

| **Vitamines :** | |
|---|---|
| . Tocophérols naturels | 5 mg |
| . Acide ascorbique | 30 mg |

| **Composant (a) :** | |
|---|---|
| . Bétatène® 20% | **15 mg**^{**1**} |

| **Composant (b) :** | |
|---|---|
| . Lycopène 5% | **60 mg**^{**2**} |

| | |
|---|---|
| ^{**1**} **correspondant à 2,86 mg de composant (a) et** | |
| ^{**2**} **correspondant à 3 mg de lycopène.** | |

Une suspension de bétatène® à 20 % comprend 200 mg/g de caroténoïdes répartis comme suit :

| | |
|---|---|
| β-carotène | 190,5 |
| α-carotène | 6 |
| zéaxanthine | 1,2 |
| cryptoxanthine | 1,4 |
| lutéine | 0,9 |

### Exemple 2: Étude de l'effet bronzant et de l'effet photoprotecteur de la composition selon l'exemple 1 (dénommée B3/L3). Comparaison avec une composition comprenant 13 mg de caroténoïdes à activité provitaminique A et 2 mg de lycopène (dénommée B13/L2).

Il s'agit d'une étude randomisée d'une durée de 8 semaines, en double-aveugle d'une composition B3/L3 selon l'invention selon l'exemple 1 et d'une composition B13/L2 comprenant 13 mg de caroténoïdes à activité provitaminique A β-carotène, monocentrique incluant un total de 20 sujets cette étude a été réalisée en Allemagne, en hiver.

### -DETAIL DE L'ETUDE

Les sujets sont sélectionnés après un examen médical destiné à vérifier leur bon état de santé général.

Des visites régulières sont nécessaires pour vérifier la tolérance et pour réaliser l'évaluation de:
- l'exposition aux UV
- mesures chromamétriques des valeurs de b (jaune), L (luminescence) et a (rouge)
- dosage direct par spectométrie à réflexion multiple de : l'hémoglobine, l'hémoglobine oxydée, l'oxygène, les mélanines et le β-carotène.

L'efficacité est déterminée par une amélioration significative de ces paramètres en les comparant avec les résultats obtenus le jour de la randomisation, avant la prise de la composition.

La relation dose-réponse est évaluée par la détermination du nombre de répondeurs dans chaque groupe.

| **- DESCRIPTION DE L'ETUDE** | | | | | |
|---|---|---|---|---|---|
| Jours | J-28 | J0 | J7 | J14 | J28 |
| Numéro de visite | V1 | V2V3 | V4V5 | V6V7 | V8V9 |
| Examen clinique | * | * | * | * | * |
| Exposition aux UV | * | * | * | * | * |
| Chromamètre | * | * | * | * | * |
| Spectrométrie | * | * | * | * | * |
| Consentement éclairé | * | | | | |
| Randomisation | * | | | | |
| Fourniture de la composition nutritiormelle à tester | * | * | * | * | * |
| Événements indésirables | * | * | * | * | * |

La composition B13/L2 consiste en capsules molles présentant la formule suivante :

| | B13/L2 |
|---|---|
| . Huile de soja hydrogénée | 40 mg |
| . Huile de blé | 95 mg |
| . Lécithine de soja | 20 mg |
| . Tocophérols naturels | 5 mg |
| . Acide ascorbique | 30 mg |
| . Bétatène® 20% | **65 mg**^{**1**} |
| . Lycopène 5% | **40 mg**^{**2**} |

| | |
|---|---|
| ^{**1**} **correspondant à 12,42 mg de caroténoïdes à activité provitaminique A et** | |
| ^{**2**} **correspondant à 2 mg de lycopène.** | |

Le traitement quotidien est l'un des suivants :
. 2 capsules de B13/L2 ou
. 2 capsules de B3/L3,
à raison de 2 capsules tous les jours entre 7 et 9 heures avec leur petit déjeuner.

### Résultats :

Les résultats obtenus sont illustrés aux Tableaux I à V ci-après et aux figures 1 à 4 et montrent que la diminution en β-carotène dans une composition selon l'invention, par rapport aux compositions de l'art antérieur permet d'obtenir une diminution significative de l'érythème obtenu après irradiation UV (Tableau III et figure 2 : évaluation de l'érythème par mesure des variations d'HbO, Tableau IV et figure 3 : évaluation de l'érythème par mesure des variations d'Hb et Tableau V et figure 4 : évaluation de l'érythème par mesure des variations d'O₂), qui de manière surprenante n'était pas possible d'obtenir avec une concentration élevée de β-carotène ; en outre, les Tableaux I et II montrent qu'une composition selon l'invention permet effectivement d'obtenir une augmentation significative du bronzage par synthèse endogène de mélanines sans caroténodermie.

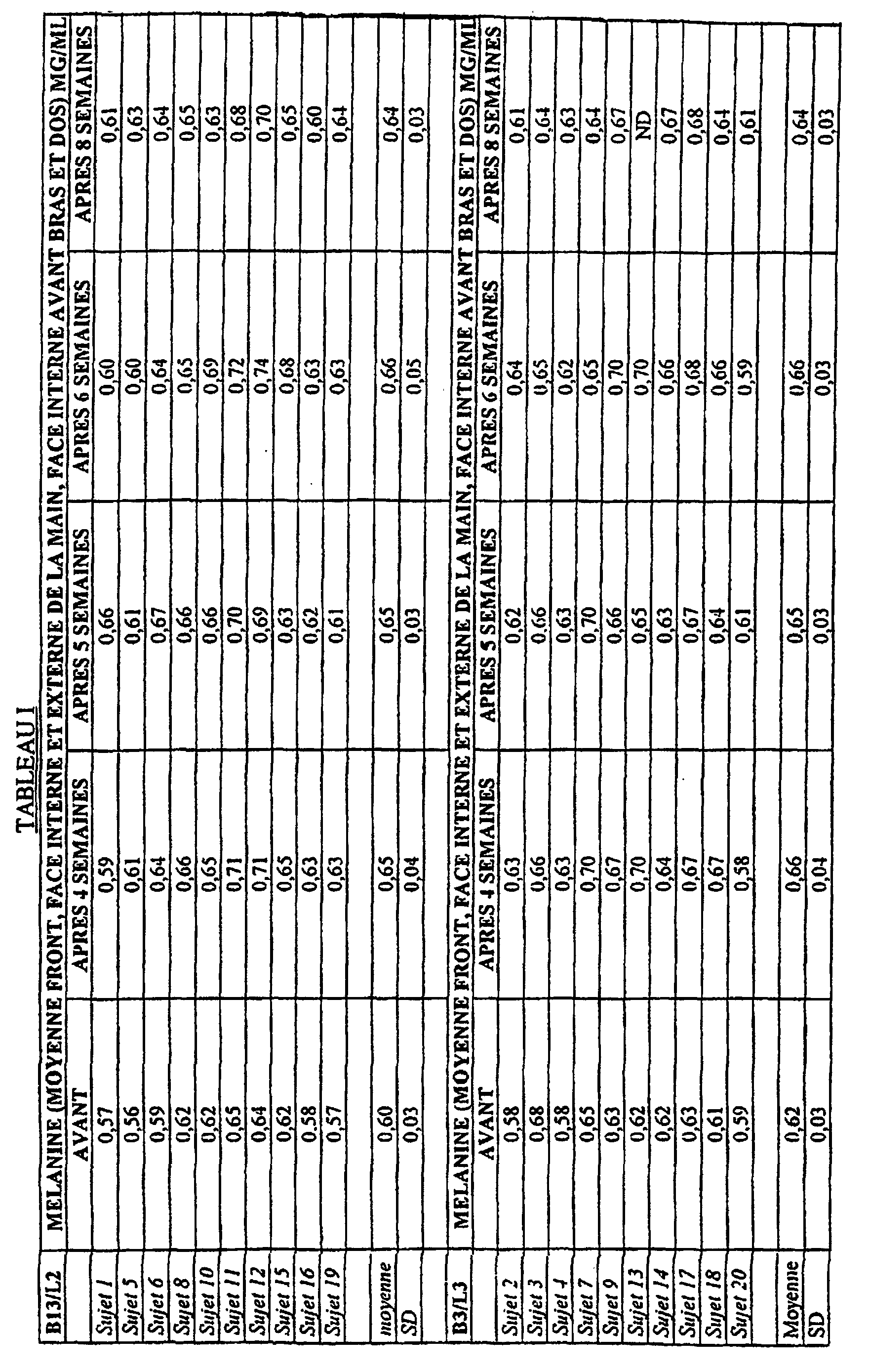

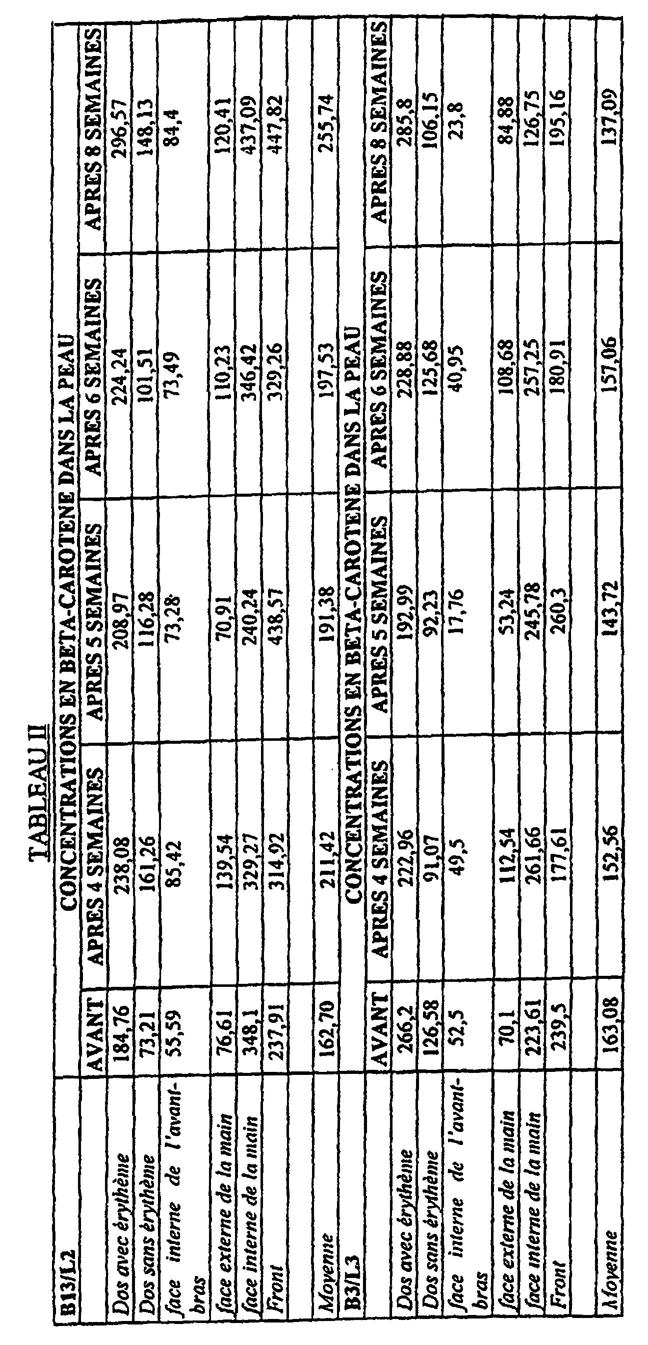

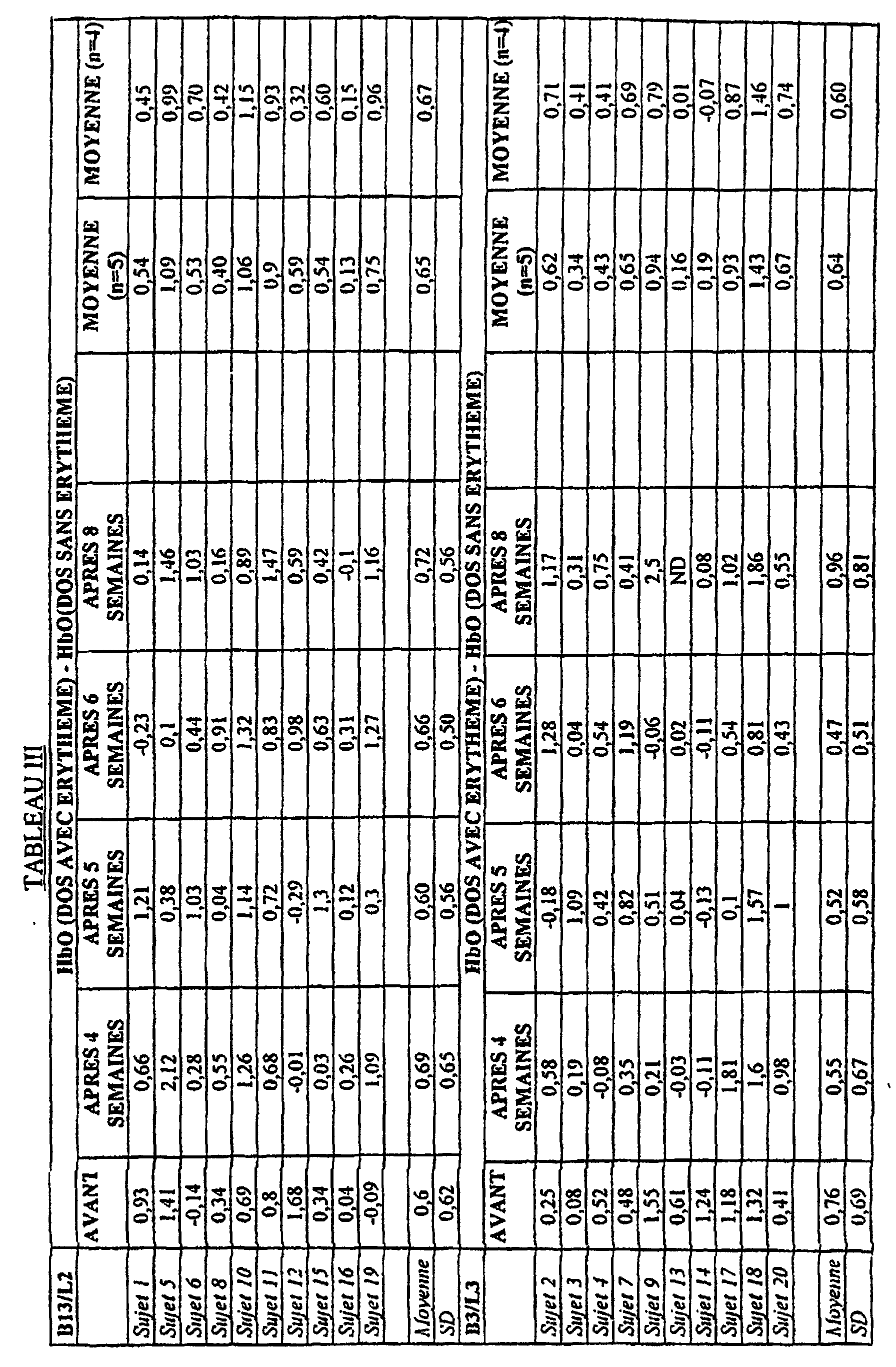

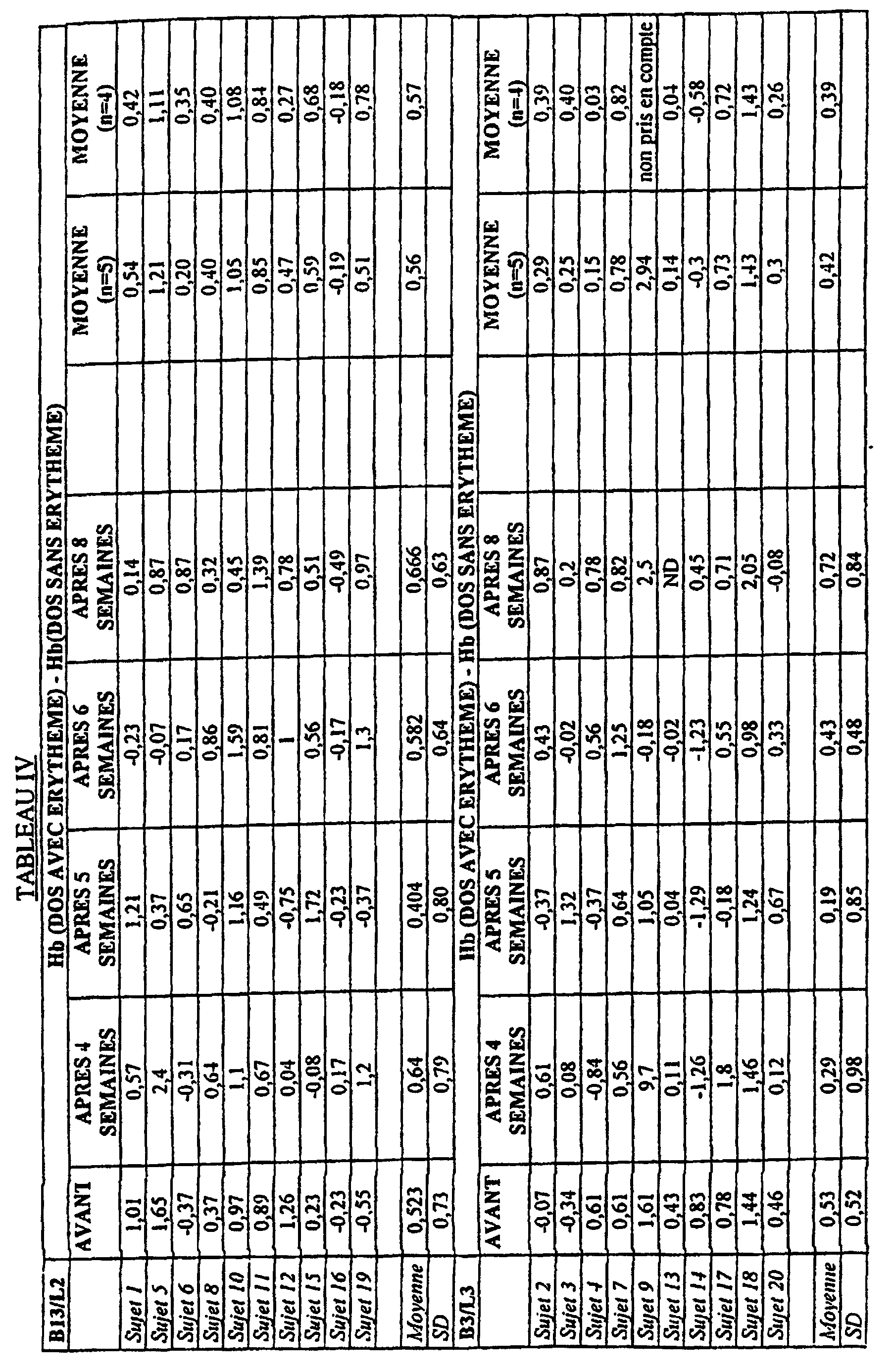

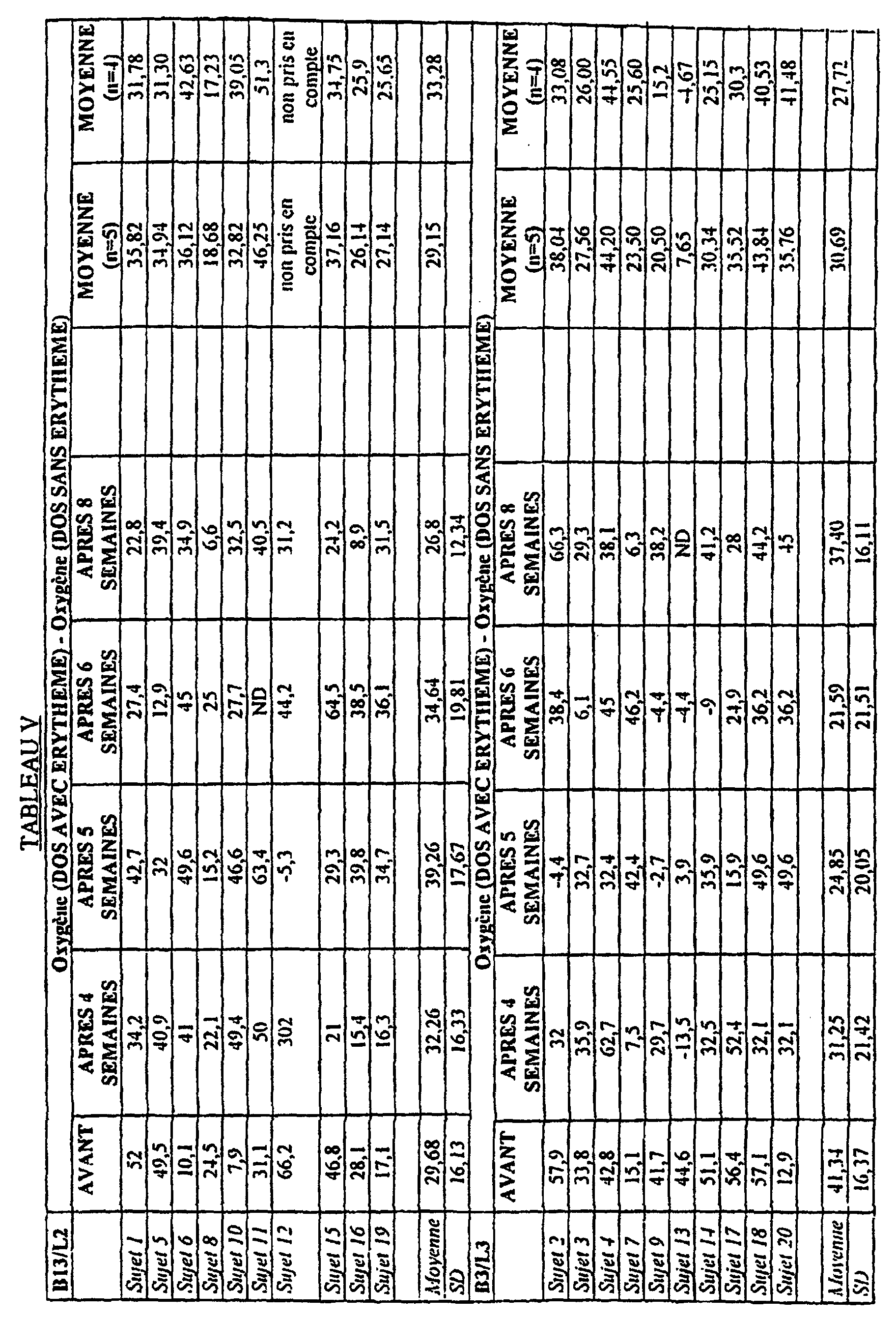

### Exemple 3 : Préparation d'une composition à administration topique selon l'invention.

| | B3/L3 |
|---|---|
| **Excipients :** | |
| . Lécithine de soja | 80 mg |
| . Huile de soja hydrogénée | 40 mg |
| . Céramides | 20 mg |

| **Vitamines :** | |
|---|---|
| . Tocophérols naturels | 5 mg |
| . Acide ascorbique | 30 mg |

| **Composant (a) :** | |
|---|---|
| .Bétatène® 20% | **15 mg**^{**1**} |

| **Composant (b) :** | |
|---|---|
| .Lycopène 5% | **60 mg**^{**2**} |

| | |
|---|---|
| ^{**1**} **correspondant à 2,86 mg de composant (a) et** | |
| ^{**2**} **correspondant à 3 mg de lycopène.** | |

## Revendications

1. Composition à visée esthétique, régulatrice de la pigmentation cutanée et adaptée à l'administration aussi bien à l'administration par voie orale qu'à l'application sur la peau, du type comprenant des caroténoïdes, **caractérisée en ce qu'**elle comprend essentiellement un composant (a) constitué d'au moins un caroténoïde d'origine naturelle à activité provitaminique A, sélectionné dans le groupe constitué par le β-carotène et l'α-carotène ou un mélange de ceux-ci et un composant (b), correspondant à au moins un caroténoïde d'origine naturelle sans activité provitaminique A, constitué par du lycopène, éventuellement associé à un autre composant (b), sélectionné dans le groupe constitué par la zéaxanthine, la cryptoxanthine et la lutéine ou un mélange de ceux-ci, les composants (a) et le lycopène étant dans un rapport compris entre 0,95/1 et 1/50.

2. Composition selon la revendication 1, **caractérisée en ce que** le composant (b) comprend du lycopène à une dose comprise entre 1 et 3 mg.

3. Composition selon la revendication 1 ou la revendication 2, **caractérisée en ce qu'**elle comprend en outre des vitamines et/ou des oligo-éléments.

4. Composition selon l'une quelconque des revendications 1 à 3, **caractérisée en ce que** lorsqu'elle est destinée à être administrée par voie orale, ladite composition comprend le composant (a) à une dose inférieure ou égale à 800 µg, exprimée en vitamine A, de préférence à une dose de 400 µg, exprimée en vitamine A.

5. Composition selon la revendication, **caractérisée en ce qu'**elle comprend en outre au moins un excipient approprié adapté à l'administration orale.

6. Composition selon la revendication 5, **caractérisée en ce qu'**elle se présente sous la forme de capsules molles.

7. Composition selon l'une quelconque des revendications 1 à 3, **caractérisée en ce qu'**elle comprend en outre au moins un excipient approprié à l'administration topique.

8. Composition selon la revendication 7, **caractérisée en ce qu'**elle se présente sous la forme de crème, de lotion, de liposomes ou de gel.

9. Méthode de traitement esthétique de l'homme pour modifier le bronzage de la peau, **caractérisée en ce qu'**elle comprend l'administration par voie orale d'une quantité appropriée journalière ne dépassant pas 800 µg, exprimée en vitamine A de composant (a), d'une composition selon l'une quelconque des revendications 1 à 6.

10. Méthode de traitement esthétique de l'homme pour modifier le bronzage de la peau, **caractérisée en ce qu'**elle comprend l'administration par voie topique d'une composition selon la revendication 7 ou la revendication 8.

11. Composition selon l'une quelconque des revendications 1-8, pour améliorer la photoprotection de la peau.

## Patentansprüche

1. Zusammensetzung für kosmetische Zwecke, die für die Hautpigmentierung regulierend ist und für eine Verabreichung, sowohl die Verabreichung auf oralem Weg als auch die Anwendung auf die Haut, angepasst ist und zu dem Typ gehört, der Carotinoide umfasst,
**dadurch gekennzeichnet,**
**dass** sie im Wesentlichen eine Komponente (a), die aus mindestens einem Carotinoid natürlichen Ursprungs mit Provitamin A-Aktivität, ausgewählt aus der Gruppe bestehend aus β-Carotin oder α-Carotin oder einem Gemisch derselben, besteht, und eine Komponente (b), die mindestens einem Carotinoid natürlichen Ursprungs ohne Provitamin A-Aktivität entspricht und aus Lycopen, gegebenenfalls assoziiert mit einer anderen Komponente (b), welche aus der Gruppe bestehend aus Zeaxanthin, Cryptoxanthin und Lutein oder einem Gemisch derselben ausgewählt ist, besteht, wobei die Komponente (a) und das Lycopen in einem Verhältnis zwischen 0,95/1 und 1/50 vorliegen, umfasst.

2. Zusammensetzung nach Anspruch 1,
**dadurch gekennzeichnet,**
**dass** die Komponente (b) Lycopen in einer Dosis zwischen 1 und 3 mg umfasst.

3. Zusammensetzung nach Anspruch 1 oder Anspruch 2,
**dadurch gekennzeichnet,**
**dass** sie außerdem Vitamine und/oder Spurenelemente umfasst.

4. zusammensetzung nach einem der Ansprüche 1 bis 3,
**dadurch gekennzeichnet,**
**dass** sie zur Verabreichung auf oralem Weg bestimmt ist, wobei die Zusammensetzung die Komponente (a) in einer Dosis von 800 µg oder weniger, ausgedrückt als Vitamin A, vorzugsweise in einer Dosis von 400 µg, ausgedrückt als Vitamin A, umfasst.

5. Zusammensetzung nach Anspruch 4,
**dadurch gekennzeichnet,**
**dass** sie außerdem mindestens ein Exzipienz, das zur oralen Verabreichung entsprechend angepasst ist, umfasst.

6. Zusammensetzung nach Anspruch 5,
**dadurch gekennzeichnet,**
**dass** sie in Form weicher Kapseln dargeboten wird.

7. Zusammensetzung nach einem der Ansprüche 1 bis 3,
**dadurch gekennzeichnet,**
**dass** sie außerdem mindestens ein Exzipienz, das zur topischen Verabreichung geeignet ist, umfasst.

8. Zusammensetzung nach Anspruch 7,
**dadurch gekennzeichnet,**
**dass** sie in Form einer Creme, einer Lotion, von Liposomen oder eines Gels dargeboten wird.

9. Verfahren zur kosmetischen Behandlung des Menschen, um die Bräune der Haut zu verändern,
**dadurch gekennzeichnet,**
**dass** es die Verabreichung einer geeigneten Tagesmenge, die 800 µg, ausgedrückt als Vitamin A von Komponente (a), einer Zusammensetzung nach einem der Ansprüche 1 bis 6 auf oralem Weg umfast.

10. Kosmetisches Behandlungsverfahren für den Menschen, um die Bräunung der Haut zu verändern,
**dadurch gekennzeichnet,**
**dass** es die Verabreichung einer Zusammensetzung nach Anspruch 7 oder Anspruch 8 auf topischem Weg umfasst.

11. Zusammensetzung nach einem der Ansprüche 1 bis 8 zur Verbesserung des Fotoschutzes der Haut.

## Claims

1. Composition for beauty purposes, regulating skin pigmentation and equally suitable for administration both by oral administration and by application to the skin, of the type comprising carotenoids, **characterized in that** it comprises essentially one component (a) consisting of at least one carotenoid of natural origin and with pro-vitamin A activity, chosen from the group consisting of β-carotene and α-carotene or a mixture thereof and one component (b) corresponding to at least one carotenoid of natural origin and without pro-vitamin A activity, consisting of lycopene, possibly in combination with another component (b) chosen from among the group consisting of zeaxanthine, cryptoxanthine and luteine or a mixture thereof, the components (a) and the lycopene being in a ratio lying between 0.95/1 and 1/50.

2. Composition according to Claim 1, **characterized in that** component (b) comprises lycopene at a dose lying between 1 and 3 mg.

3. Composition according to Claim 1 or Claim 2, **characterized in that** it comprises in addition vitamins and/or oligo-elements (trace elements).

4. Composition according to any of the Claims 1 to 3, **characterized in that** it is intended for administration by the oral route, the said composition comprising component (a) at a dose less than or equal to 800 µg expressed as vitamin A, preferably at a dose of 400 µg expressed as vitamin A.

5. Composition according to the Claim, **characterized in that** it comprises in addition at least one appropriate excipient suitable for oral administration.

6. Composition according to Claim 5, **characterized in that** it is presented in the form of soft capsules.

7. Composition according to any of the Claims 1 to 3, **characterized in that** it comprises in addition at least one excipient suitable for topical administration.

8. Composition according to Claim 7, **characterized in that** it is presented in the form of cream, lotion, liposomes or gel.

9. Human beauty treatment method to modify the tanning of the skin, **characterized in that** it comprises administering by the oral route an appropriate daily quantity not exceeding 800 µg expressed as vitamin A of component (a), of a composition according to any of the Claims 1 to 6.

10. Human beauty treatment method to modify the tanning of the skin, **characterized in that** it comprises administration by the topical route of a composition according to Claim 7 or Claim 8.

11. Composition according to any of the Claims 1 - 8 to improve the photo-protection of the skin.
